# FASCICULE DE BREVET EUROPEEN

(11) **EP 2 374 503 B1**
(45) Date de publication et mention de la délivrance du brevet: **11.07.2012**
(21) Numéro de dépôt: 10178589.7
(22) Date de dépôt: 23.09.2010
(51) Int. Cl.: A61N 1/36

(54) **Dispositif médical implantable actif de stimulation vagale à optimisation du remplissage ventriculaire**
Aktive implantierbare medizinische Vorrichtung für Vagusnervstimulation mit Optimisierung der Ventrikelfüllung
Active implantable medical device for vagal stimulation with optimised ventricular filling

(30) Priorité: 08.04.2010 FR 1052649
(43) Date de publication de la demande: 12.10.2011
(73) Titulaire: Sorin CRM SAS, 92143 Clamart Cedex (FR)
(72) Inventeur: Limousin, Marcel M., 75014, PARIS (FR)
(74) Mandataire: Dupuis-Latour, Dominique

(56) Documents cités:
- EP-A1- 1 433 496
- US-A- 5 916 239
- US-A1- 2006 100 668
- US-A1- 2006 264 764
- US-A1- 2008 119 898
- US-A1- 2008 125 827
- US-A1- 2008 281 368

## Description

L'invention concerne les "dispositifs médicaux implantables actifs" tels que définis par la directive 90/385/CEE du 20 juin 1990 du Conseil des communautés européennes.

Elle concerne plus particulièrement les implants comportant des moyens de stimulation vagale. Cette stimulation permet, entre autres indications, un contrôle de la pression sanguine par action sur le système sympathique.

Ces dispositifs comportent généralement un générateur implanté, relié à une sonde portant des électrodes disposées en contact avec le nerf vague au niveau de l'artère carotide afin de délivrer sur ce nerf des impulsions électriques avec un niveau d'énergie contrôlée. La stimulation du nerf vague par des impulsions électriques permet de contrôler la pression sanguine, et peut s'avérer un traitement de choix dans l'hypertension réfractaire aux médicaments.

Un tel dispositif comprenant des moyens de stimulation vagale est décrit par exemple dans le US 7 123 961 B1. Le générateur est pourvu d'un système de gestion de l'énergie de stimulation neuro-vagale permettant de gérer la contractilité du myocarde (inotropie), la fréquence cardiaque, ou la conduction auriculo-ventriculaire (dromotropie). Ces différents paramètres sont contrôlés en fonction des résultats d'une analyse des composantes du rythme cardiaque, notamment la fréquence cardiaque, le délai PR et la durée du complexe QRS.

Les US 2008/0125827 A1, US 2008/0119898 A1 et US 2006/0111668 A1 décrivent d'autres dispositifs similaires.

Le point de départ de l'invention réside dans la constatation clinique de ce que l'hypertension réfractaire aux médicaments est une pathologie très présente chez les patients souffrant d'insuffisance cardiaque, notamment ceux souffrant d'insuffisance cardiaque dite "insuffisance diastolique". Dans cette dernière famille de patients, la fonction gauche du coeur est préservée (d'où également la dénomination d'insuffisance cardiaque "à fonction systolique préservée"), la pathologie se caractérisant pendant la diastole par une phase de remplissage anormalement réduite du fait que ces patients présentent une hypertrophie ventriculaire gauche qui ne permet pas un remplissage optimal.

La présence d'une hypertension est un obstacle supplémentaire allant à l'encontre de ce remplissage. Or, si l'on traite l'hypertension par stimulation vagale, l'une des contraintes de cette technique est que son efficacité est dépendante de l'énergie de stimulation : plus la tension délivrée par l'appareil est élevée, plus la réduction de la pression sanguine est importante. Toutefois, une énergie trop importante limitera significativement la durée de vie de l'implant, en raison d'une consommation excessive.

L'un des buts de l'invention est de proposer un dispositif implantable pourvu de moyens de stimulation vagale qui soient particulièrement adaptés aux patients à la fois insuffisants cardiaques à fonction systolique préservée (patients présentant une insuffisance diastolique) et présentant en même temps une hypertension non contrôlée.

Plus précisément, l'invention a pour but de proposer un tel dispositif qui n'utilise pas de capteur de pression grâce à une technique permettant de surveiller la pression sanguine de façon indirecte en évaluant le temps de remplissage du ventricule gauche à partir de paramètres obtenus par un capteur de mesure des paramètres hémodynamiques du cycle cardiaque. Ce temps de remplissage, ou temps de remplissage diastolique, est l'intervalle de temps séparant la fermeture de la valvule aortique de la fermeture de la valvule mitrale. Comme les patients insuffisants cardiaques à fonction systolique préservée présentent une réduction significative de ce temps de remplissage diastolique, la thérapie de stimulation vagale pourra être contrôlée en fonction de ce paramètre.

Comme on le verra par la suite, ce capteur de mesure des paramètres hémodynamiques du cycle cardiaque peut être avantageusement un capteur d'accélération endocardiaque EA (*Endocardial Acceleration*). Un tel capteur permet non seulement la mesure indirecte des variations dP/dt de la pression dans le ventricule gauche, mais également la détermination de divers instants caractéristiques des phases systolique et diastolique en générant des marqueurs correspondant à ces différents instants. L'utilisation d'un tel capteur présente subsidiairement l'avantage de pouvoir évaluer la contractilité du myocarde, à partir des variations de pression dP/dt. La stimulation vagale pourra être ainsi ajustée de manière à optimiser le temps de remplissage en surveillant également la contractilité de manière à éviter l'effet pervers dit "effet inotrope négatif' qui se caractérise par une baisse de la contractilité du ventricule gauche du fait de la stimulation du nerf vague.

Plus précisément, l'invention propose un dispositif médical implantable actif du type général divulgué notamment par le US 2008/0125827 A1 précité, c'est-à-dire comprenant : des moyens de stimulation vagale, aptes à délivrer au nerf vague du patient porteur du dispositif, de façon répétée au cours de cycles cardiaques successifs, des impulsions présentant un niveau d'énergie ajusté ; un capteur de mesure des paramètres hémodynamiques du cycle cardiaque, apte à délivrer un paramètre temporel représentatif du temps de remplissage ventriculaire ; et des moyens d'ajustement de l'énergie de stimulation vagale, opérant dynamiquement de façon répétée sur plusieurs cycles cardiaques et comprenant des moyens aptes à faire varier le niveau de l'énergie de stimulation au cours de cycles cardiaques successifs.

De façon caractéristique de l'invention, les moyens aptes à faire varier le niveau de l'énergie de stimulation au cours de cycles cardiaques successifs sont également aptes à évaluer les variations corrélatives du temps de remplissage, et le dispositif comprend en outre des moyens pour établir l'énergie de stimulation à un niveau ajusté maximisant ledit temps de remplissage.

Le capteur de mesure des paramètres hémodynamiques du cycle cardiaque peut notamment être un capteur d'accélération endocardiaque. Les moyens d'ajustement de l'énergie de stimulation vagale comprennent alors : des moyens aptes à isoler dans le signal délivré par le capteur d'accélération endocardiaque au moins une composante correspondant à un pic d'accélération endocardiaque ; des moyens aptes à opérer une analyse morphologique de ladite composante ; et des moyens aptes à dériver ledit paramètre temporel représentatif du temps de remplissage ventriculaire des résultats de ladite analyse morphologique.

De préférence, les moyens d'ajustement de l'énergie de stimulation vagale comprennent également : des moyens pour comparer le temps de remplissage mesuré à un seuil prédéterminé ; et des moyens pour limiter ledit niveau ajusté au niveau d'énergie de stimulation courant dès que ledit seuil est atteint.

Dans une mise en oeuvre préférentielle, le capteur de mesure des paramètres hémodynamiques du cycle cardiaque est également apte à délivrer un paramètre hémodynamique représentatif de la contractilité du ventricule gauche, et les moyens d'ajustement de l'énergie de stimulation vagale comprennent également : des moyens pour évaluer les variations du paramètre hémodynamique au cours des cycles cardiaques successifs ; et des moyens pour limiter ledit niveau ajusté au niveau d'énergie de stimulation courant en cas d'augmentation du paramètre hémodynamique faisant suite à une augmentation du niveau d'énergie de stimulation.

En particulier, avec un capteur d'accélération endocardiaque, les moyens de mesure des paramètres hémodynamiques peuvent comprendre : des moyens aptes à isoler dans le signal délivré par le capteur d'accélération endocardiaque au moins une composante correspondant à un pic d'accélération endocardiaque ; et des moyens aptes à dériver de ladite composante l'indice hémodynamique.

Le dispositif comprenant de préférence des moyens pour activer les moyens d'ajustement de l'énergie de stimulation vagale à intervalles réguliers prédéterminés, et/ou pour détecter des changements d'état prédéterminés du patient et activer les moyens d'ajustement de l'énergie de stimulation vagale sur détection d'un changement d'au moins un état du patient, ces changements d'état étant notamment des changements du groupe formé par : présence ou absence d'arythmies ; transitions entre phases de repos, d'effort et de sommeil.

Le capteur de mesure des paramètres hémodynamiques du cycle cardiaque peut être un capteur du groupe formé par : capteur d'accélération endocavitaire ou épicardique ; capteur de mouvement d'une paroi du myocarde ; capteur de pression intracardiaque ; capteur de bioimpédance intracardiaque ; capteur de saturation d'oxygène par mesure optique ; capteur de mesure de variation de volume par ultrasons.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une vue schématique d'un dispositif de stimulation vagale implanté chez un patient, avec sa sonde endocardiaque et ses électrodes de stimulation du nerf vague.
La Figure 2 est un organigramme expliquant la manière dont est ajustée l'énergie de stimulation vagale selon une première forme de mise en oeuvre de l'invention.
La Figure 3 est un organigramme homologue de celui de la figure 2, pour une seconde forme de mise en oeuvre de l'invention.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* et *Paradym* produits et commercialisés par Sorin CRM, Clamart, France.

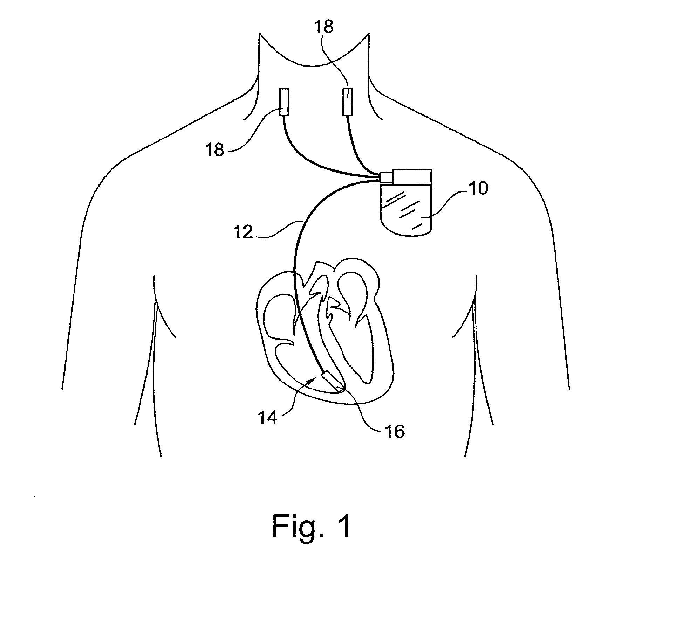

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail. Sur la Figure 1, la référence 10 désigne le boîtier du générateur implanté chez le patient, relié à une sonde 12 comportant à son extrémité distale 14 un capteur 16 de mesure des paramètres hémodynamiques du cycle cardiaque. La sonde 12 peut également porter diverses électrodes de recueil de signaux de dépolarisation du coeur et/ou de stimulation cardiaque, mais il s'agit là d'éléments qui ne font pas l'objet de la présente invention, qui ne concerne que la stimulation vagale, et non la stimulation cardiaque.

Le capteur 16 de mesure des paramètres hémodynamiques du cycle cardiaque a pour objet de mesurer des variations du volume du ventricule gauche, et/ou du mouvement des fibres musculaires de ce ventricule, pendant les différentes phases du cycle cardiaque.

Ces différentes phases comprennent : pré-éjection, contraction isovolumique, éjection systolique, relaxation isovolumique et enfin remplissage de la cavité ventriculaire.

L'invention s'intéresse particulièrement au temps de remplissage du ventricule gauche ou temps de remplissage diastolique, qui est la durée comprise entre l'instant de la fermeture de la valve aortique et celui de la fermeture de la valve mitrale. Ce temps de remplissage est généralement désigné FT ou DFT (*Diastolic Filling Time*).

Les instants caractéristiques marquant les différentes phases du cycle cardiaque peuvent être déterminés au moyen d'une technique telle que celle décrite notamment dans le EP 2 092 885 A1 (Sorin CRM, anciennement ELA Medical). Ce document décrit une technique d'analyse morphologique d'un signal d'accélération endocardiaque EA (*Endocardial Acceleration*) délivré par un accéléromètre en contact avec le muscle cardiaque, notamment un capteur intégré à une sonde endocavitaire. Les données fournies par un tel capteur reflètent en effet très précisément et en temps réel les phénomènes concourant au fonctionnement mécanique du coeur et permettent ainsi, après filtrage et analyse, de fournir des marqueurs temporels de la diastole ainsi que d'autres indices de performance hémodynamique du myocarde. Les marqueurs temporels obtenus sont corrélés aux instants d'ouverture et de fermeture des valves aortique, mitrale, pulmonaire et/ou tricuspide, et le temps de remplissage est évalué à partir de la durée comprise entre la fermeture de la valve aortique et la fermeture de la valve mitrale. Ces paramètres peuvent être déterminés en temps réel, battement après battement, ce qui permet d'estimer à chaque instant les performances hémodynamiques du coeur et adapter sans délai la thérapie appliquée au patient.

De façon générale, les capteurs de mesure des paramètres hémodynamiques du cycle cardiaque sont des capteurs permettant d'estimer les variations de contractilité du myocarde, corrélées aux augmentations de la pression sanguine. Ils se distinguent des capteurs d'activité (capteurs d'accélération par exemple) et des capteurs métaboliques (capteurs de ventilation-minute par exemple), qui ne sont destinés qu'à diagnostiquer la présence ou non d'un exercice par le patient et à quantifier ses besoins métaboliques, par exemple pour adapter la fréquence cardiaque de stimulation en fonction du niveau détecté. Les capteurs hémodynamiques peuvent notamment donner une indication de la tolérance hémodynamique du patient par rapport à certains événements, en particulier, comme on le verra plus bas, la tolérance à une modification des paramètres de la stimulation vagale.

Dans les exemples que l'on décrira ici, on prendra pour exemple de capteur de mesure des paramètres hémodynamiques du cycle cardiaque un capteur endocavitaire d'accélération endocardiaque (capteur EA). Ces exemples ne sont toutefois aucunement limitatifs, et l'invention peut être mise en oeuvre avec d'autres capteurs de mesure des paramètres hémodynamiques du cycle cardiaque tels que : capteur d'accélération épicardique (et non plus endocavitaire), capteur de mouvement d'une paroi du myocarde, capteur de pression intracardiaque permettant de déterminer la différence de pression Δpp entre la pression systolique et la pression diastolique, capteur de bioimpédance intracardiaque, capteur de saturation d'oxygène par mesure optique, capteur de mesure de variation de volume par ultrasons, etc.

Pour diverses descriptions de tels capteurs, on pourra se référer notamment aux documents suivants :
- pour un capteur d'accélération endocardiaque de type capteur EA : le EP 0 515 319 A1 (Sorin Biomedica Cardio SpA), qui décrit la manière de recueillir un signal d'accélération endocardiaque au moyen d'une sonde endocavitaire pourvue d'une électrode distale de stimulation implantée au fond du ventricule et intégrant un micro-accéléromètre permettant de mesurer l'accélération endocardiaque, et le EP 0 655 260 A1 (Sorin Biomedica Cardio SpA), qui décrit une manière de traiter le signal d'accélération endocardiaque mesuré pour en dériver notamment la valeur des pics d'accélération endocardiaque correspondant aux deux bruits majeurs qu'il est possible de reconnaître dans chaque cycle d'un coeur sain ;
- pour un capteur de bioimpédance transvalvulaire (entre oreillette et ventricule situés d'un même côté du coeur) : le EP 1 116 497 A1 (Sorin CRM, anciennement ELA Medical), qui décrit une mesure dynamique de la bioimpédance (BioZ) afin d'évaluer les volumes diastolique et systolique et obtenir ainsi une indication du débit cardiaque et donc de la fraction d'éjection ;
- pour un capteur de bioimpédance transseptale (entre un site situé d'un côté du coeur et un site situé de l'autre côté) : le EP 1 138 346 A1 (Sorin CRM, anciennement ELA Medical), qui décrit un autre type de mesure permettant de délivrer également une valeur représentative de la fraction d'éjection.

Dans le cas d'un capteur EA, le signal d'accélération endocardiaque recueilli au cours d'un cycle cardiaque forme deux composantes principales, correspondant aux deux bruits majeurs du coeur (sons S1 et S2 du phonocardiogramme) qu'il est possible de reconnaître dans chaque cycle :
- la première composante d'accélération endocardiaque ("EA1"), dont les variations d'amplitude sont étroitement liées aux variations de la pression dans le ventricule (l'amplitude maximale crête-à-crête de cette composante EA1, appelée PEA1, étant plus précisément corrélée au maximum positif de la variation de pression dP/dt dans le ventricule gauche) et peuvent donc constituer un paramètre représentatif de la contractilité du myocarde, elle-même liée au niveau d'activité du système sympathique ;
- la seconde composante d'accélération endocardiaque ("EA2") qui, quant à elle, survient pendant la phase de relaxation ventriculaire isovolumique. Cette seconde composante, qui est produite principalement par la décélération brusque de la masse sanguine en mouvement dans l'aorte après fermeture des valves aortique et pulmonaire, constitue un paramètre représentatif de la pression sanguine périphérique au début de la diastole.

Le signal contient parfois une ou deux autres composantes, EA3 et EA4, correspondant aux sons S3 et S4 du phonocardiogramme.

Outre le capteur 16 de mesure des paramètres hémodynamiques du cycle cardiaque, le générateur 10 est également relié à des électrodes 18 de stimulation du nerf vague, notamment des électrodes placées en configuration bilatérale au niveau de l'artère carotide, de manière à contrôler la pression cardiaque du patient.

La stimulation du système vagal est en effet connue pour réduire la pression chez le patient hypertendu.

Essentiellement, l'invention vise à contrôler cette stimulation vagale de manière à optimiser le temps de remplissage du ventricule gauche (paramètre FT).

On considère généralement que l'optimum de ce temps de remplissage est atteint pour FT > 40 % (le temps de remplissage est généralement exprimé de manière relative, en pourcentage de la durée complète d'un cycle (durée RR). Cette valeur sera appelée "valeur cible".

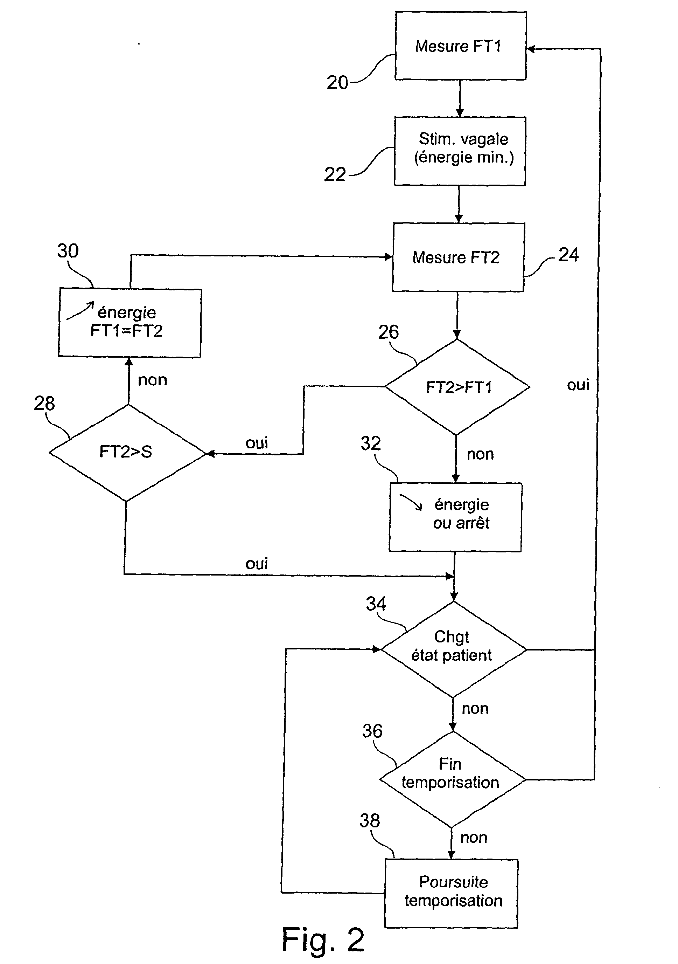

On a illustré sur la Figure 2 une première mise en oeuvre de la technique de l'invention.

La première étape (étape 20) consiste à mesurer la valeur courante FT1 du temps de remplissage en l'absence de stimulation vagale. La stimulation vagale est ensuite appliquée (étape 22), avec une énergie minimale pour les impulsions délivrées aux électrodes en contact avec le nerf vague.

Le temps de remplissage est à nouveau mesuré (étape 24), donnant une valeur FT2 qui est comparée (étape 26) à la précédente valeur FT1.

Si FT2 > FT1 au test 26, c'est-à-dire si la stimulation vagale a conduit à une augmentation du temps de remplissage, la valeur FT2 est comparée à la valeur cible S (étape 28), typiquement S = 40 % de la durée d'un cycle cardiaque :
- si la valeur cible S n'est pas atteinte, l'énergie de stimulation vagale est augmentée d'un pas et FT2 devient la nouvelle valeur courante FT1 du temps de remplissage (étape 30), puis le temps de remplissage est mesuré avec cette nouvelle valeur d'énergie de stimulation (retour à l'étape 24) et comparé à la valeur précédente (étape 26), et ainsi de suite ;
- si la valeur cible a été atteinte ou ne croît plus, ceci signifie que le remplissage est satisfaisant, et qu'il n'est donc pas nécessaire d'augmenter encore l'énergie de stimulation (une telle augmentation non seulement obérerait l'autonomie de la batterie de l'implant, mais risquerait en outre d'introduire un effet pervers de diminution de la contractilité du ventricule). L'énergie de stimulation vagale est maintenue à son niveau courant, qui est donc celui permettant d'obtenir le temps de remplissage idéal (valeur cible), et il est mis fin au processus itératif.

Si le test de l'étape 26 n'avait pas révélé d'augmentation du temps de remplissage consécutive à l'augmentation de l'énergie de stimulation vagale, ceci signifie que l'optimum a été dépassé. L'énergie est alors diminuée d'un pas (étape 32) pour revenir à sa valeur précédente ou bien, si le test faisait suite à une stimulation à énergie minimale (étape 22), la stimulation vagale est purement et simplement arrêtée (car ceci signifie que, loin de produire une amélioration du temps de remplissage, la stimulation vagale induit au contraire une diminution de ce paramètre).

Une fois atteinte la valeur optimale, le procédé d'ajustement itératif du niveau d'énergie de stimulation est interrompu et l'énergie de stimulation est stabilisée à la dernière valeur atteinte.

Cette situation est maintenue jusqu'à ce qu'un changement d'état du patient soit détecté (étape 34) ou à la fin d'un délai prédéterminé (étape 36), par exemple un délai de temporisation de six heures. Dans le cas contraire la temporisation est poursuivie (étape 38), par comptage du temps écoulé ou comptage d'un nombre de cycles cardiaques depuis le dernier ajustement de l'énergie de stimulation.

Sur détection d'un changement d'état ou en fin de la temporisation, le processus que l'on vient d'exposer plus haut est relancé.

Le changement d'état du patient est détecté par des capteurs conventionnes, par exemple un capteur d'accélération (capteur G) intégré au boîtier du générateur, un capteur de ventilation-minute (capteur MV), ou même par le capteur d'accélération endocardiaque, ces différents capteurs pouvant détecter par analyse du signal le changement de certaines circonstances telles que : effort du patient, repos prolongé, sommeil, survenue d'arythmies, etc.

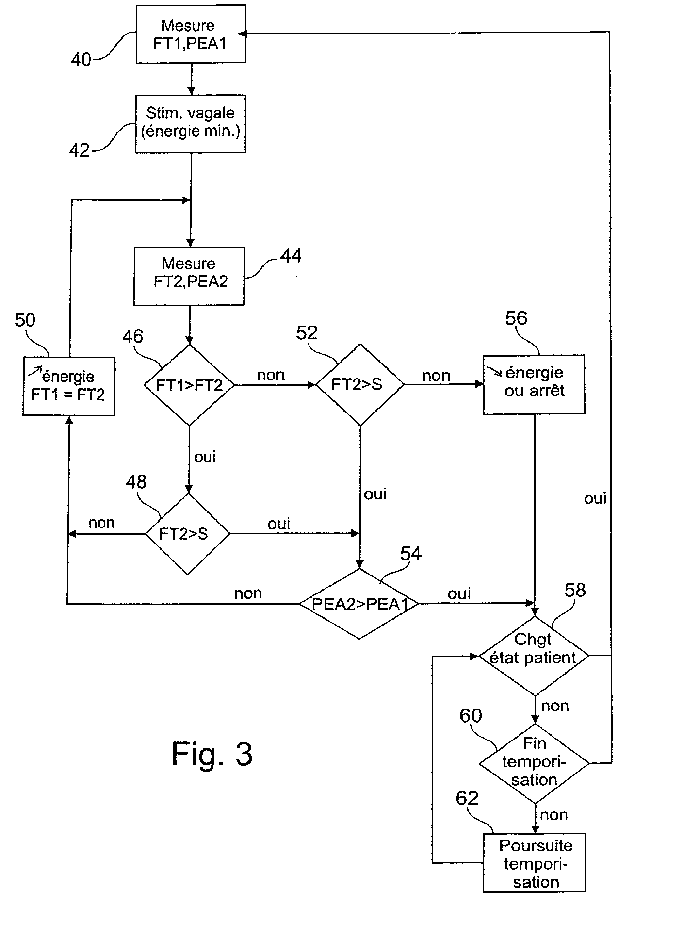

On a illustré sur la Figure 3 une deuxième mise en oeuvre de la technique de l'invention, incluant un perfectionnement du processus que l'on vient de décrire.

En effet, dans le cas de l'organigramme de la Figure 2, le seul paramètre testé est le temps de remplissage. Dans la mise en oeuvre de la Figure 3, le système cherche non seulement à obtenir le temps de remplissage FT le plus long possible, mais teste également les modifications éventuelles de la contractilité du myocarde résultant de la variation de l'énergie de stimulation vagale.

En effet, on peut constater parfois une baisse de la contractilité du ventricule gauche résultant de la stimulation du nerf vague (effet inotrope négatif) et il est important de ne pas dégrader ce paramètre lors de l'ajustement de l'énergie de stimulation vagale par un niveau excessif d'énergie de stimulation.

Ce mode de réalisation tire parti du fait qu'un capteur d'accélération endocardiaque EA permet non seulement d'obtenir des marqueurs temporels repérant les instants caractéristiques du cycle et donc de calculer le temps de remplissage, mais également d'obtenir un paramètre non temporel représentatif de la contractilité du myocarde. Ce paramètre est typiquement obtenu par la mesure du maximum pic à pic du signal EA délivré par le capteur 16.

Les étapes 40, 42 et 44 de la Figure 3 sont semblables aux étapes 20, 22 et 24 de la Figure 2 décrites plus haut, avec en outre aux étapes 40 et 44 la mesure non seulement du temps de remplissage FT1 et FT2 avant et après modification de l'énergie de stimulation vagale, mais également mesure de l'amplitude du premier pic d'accélération endocardiaque avant (PEA1) et après (PEA2) modification de cette énergie.

De même, les étapes 46, 48 et 50 de la Figure 3 sont semblables aux étapes 26, 28 et 30 de la Figure 2.

A l'étape 46, si le test n'a pas montré d'amélioration du temps de remplissage FT suite à l'augmentation de l'énergie de stimulation, alors le temps de remplissage est à nouveau testé par rapport à la valeur cible S (étape 52). Si la valeur cible est atteinte, ceci signifie que l'augmentation de l'énergie n'a pas amélioré la situation ; cette énergie est donc réduite, ou le cas échéant la stimulation est arrêtée (étape 56), de la même façon qu'à l'étape 32 de la Figure 2.

A l'étape 52, si le test indique que la valeur cible S est atteinte, on teste à l'étape 54 si la contractilité a augmenté, en comparant les mesures PEA1 et PEA2 du premier pic d'amplitude respectivement avant et après augmentation de l'énergie de stimulation vagale :
- si la contractilité a augmenté (PEA2 > PEA1), le processus itératif est achevé, l'énergie de stimulation est maintenue à son niveau courant et le système attend soit un changement d'état du patient (étape 58), soit la fin d'un délai de temporisation (étapes 60, 62) pour recommencer le test d'ajustement de l'énergie ;
- si, en revanche, le test de l'étape 54 révèle une diminution de la contractilité (PEA2 < PEA1), alors l'énergie est rétablie à son niveau précédent (étape 50) de manière à retourner à la situation antérieure, la réduction de l'énergie de stimulation permettant de pallier la dégradation de la contractilité.

## Revendications

1. Un dispositif médical implantable actif, comprenant :
- des moyens de stimulation vagale (10, 18), aptes à délivrer au nerf vague du patient porteur du dispositif, de façon répétée au cours de cycles cardiaques successifs, des impulsions présentant un niveau d'énergie ajusté ;
- un capteur (16) de mesure des paramètres hémodynamiques du cycle cardiaque, apte à délivrer un paramètre temporel représentatif du temps de remplissage ventriculaire ; et
- des moyens d'ajustement de l'énergie de stimulation vagale, opérant dynamiquement de façon répétée sur plusieurs cycles cardiaques et comprenant des moyens aptes à faire varier (30, 32 ; 50, 56) le niveau de l'énergie de stimulation au cours de cycles cardiaques successifs, dispositif **caractérisé en ce que** :
- lesdits moyens aptes à faire varier le niveau de l'énergie de stimulation au cours de cycles cardiaques successifs sont également aptes à évaluer (26 ; 46) les variations corrélatives du temps de remplissage (FT1, FT2), et
- le dispositif comprend en outre des moyens pour établir l'énergie de stimulation à un niveau ajusté maximisant ledit temps de remplissage.

2. Le dispositif de la revendication 1, dans lequel :
- le capteur (16) de mesure des paramètres hémodynamiques du cycle cardiaque est un capteur d'accélération endocardiaque, et
- les moyens d'ajustement de l'énergie de stimulation vagale comprennent :
· des moyens aptes à isoler dans le signal délivré par le capteur d'accélération endocardiaque au moins une composante correspondant à un pic d'accélération endocardiaque ;
· des moyens aptes à opérer une analyse morphologique de ladite composante ; et
· des moyens aptes à dériver ledit paramètre temporel représentatif du temps de remplissage ventriculaire des résultats de ladite analyse morphologique.

3. Le dispositif de la revendication 1, dans lequel les moyens d'ajustement de l'énergie de stimulation vagale comprennent également :
· des moyens pour comparer (28 ; 48, 52) le temps de remplissage mesuré à un seuil prédéterminé (S) ; et
· des moyens pour limiter ledit niveau ajusté au niveau d'énergie de stimulation courant dès que ledit seuil est atteint.

4. Le dispositif de la revendication 1, dans lequel :
- le capteur de mesure des paramètres hémodynamiques du cycle cardiaque est également apte à délivrer un paramètre hémodynamique représentatif de la contractilité du ventricule gauche, et
- les moyens d'ajustement de l'énergie de stimulation vagale comprennent également :
· des moyens pour évaluer (54) les variations du paramètre hémodynamique (PEA1, PEA2) au cours des cycles cardiaques successifs ; et
· des moyens pour limiter ledit niveau ajusté au niveau d'énergie de stimulation courant en cas d'augmentation du paramètre hémodynamique faisant suite à une augmentation du niveau d'énergie de stimulation.

5. Le dispositif de la revendication 4, dans lequel :
- le capteur (16) de mesure des paramètres hémodynamiques du cycle cardiaque est un capteur d'accélération endocardiaque, et
- les moyens de mesure des paramètres hémodynamiques comprennent :
· des moyens aptes à isoler dans le signal délivré par le capteur d'accélération endocardiaque au moins une composante correspondant à un pic d'accélération endocardiaque ; et
· des moyens aptes à dériver de ladite composante l'indice hémodynamique (PEA1, PEA2).

6. Le dispositif de la revendication 1, comprenant des moyens pour activer (36, 38 ; 60, 62) les moyens d'ajustement de l'énergie de stimulation vagale à intervalles réguliers prédéterminés.

7. Le dispositif de la revendication 1, comprenant :
- des moyens pour détecter (34 ; 58) des changements d'état prédéterminés du patient ; et
- des moyens pour activer les moyens d'ajustement de l'énergie de stimulation vagale sur détection d'un changement d'au moins un état du patient.

8. Le dispositif de la revendication 7, dans lequel les changements d'état prédéterminés sont des changements du groupe formé par : présence ou absence d'arythmies ; transitions entre phases de repos, d'effort et de sommeil.

9. Le dispositif de la revendication 1, dans lequel le capteur (16) de mesure des paramètres hémodynamiques du cycle cardiaque est un capteur du groupe formé par : capteur d'accélération endocavitaire ou épicardique ; capteur de mouvement d'une paroi du myocarde ; capteur de pression intracardiaque ; capteur de bioimpédance intracardiaque ; capteur de saturation d'oxygène par mesure optique ; capteur de mesure de variation de volume par ultrasons.

## Claims

1. Active implantable medical device, comprising:
- vagal stimulation means (10, 18), designed to deliver to the vagal nerve of the patient wearing the device, repeatedly during successive cardiac cycles, pulses having an adjusted energy level;
- a sensor (16) for measuring the haemodynamic parameters of the cardiac cycle, designed to deliver a temporal parameter representative of the ventricular filling time; and
- means for adjusting the vagal stimulation energy, operating dynamically in a repetitive manner over a number of cardiac cycles and comprising means designed to vary (30, 32; 50, 56) the level of the stimulation energy during successive cardiac cycles,
which device is **characterized in that**:
- said means designed to vary the level of the stimulation energy during successive cardiac cycles are also designed to evaluate (26; 46) the correlative variations of the filling time (FT1, FT2), and
- the device also comprises means for setting the stimulation energy at an adjusted level maximizing said filling time.

2. Device according to Claim 1, in which:
- the sensor (16) for measuring the haemodynamic parameters of the cardiac cycle is an endocardiac acceleration sensor, and
- the means for adjusting the vagal stimulation energy comprise:
· means designed to isolate, in the signal delivered by the endocardiac acceleration sensor, at least one component corresponding to an endocardiac acceleration spike;
· means designed to conduct a morphological analysis of said component; and
· means designed to derive said temporal parameter representative of the ventricular filling time from the results of said morphological analysis.

3. Device according to Claim 1, in which the means for adjusting the vagal stimulation energy also comprise:
· means for comparing (28; 48, 52) the measured filling time to a predetermined threshold (S); and
· means for limiting said adjusted level to the current stimulation energy level as soon as said threshold is reached.

4. Device according to Claim 1, in which:
- the sensor for measuring the haemodynamic parameters of the cardiac cycle is also designed to deliver a haemodynamic parameter representative of the contractility of the left ventricle, and
- the means for adjusting the vagal stimulation energy also comprise:
· means for evaluating (54) the variations of the haemodynamic parameter (PEA1, PEA2) during successive cardiac cycles; and
· means for limiting said adjusted level to the current stimulation energy level should the haemodynamic parameter increase following an increase in the stimulation energy level.

5. Device according to Claim 4, in which:
- the sensor (16) for measuring the haemodynamic parameters of the cardiac cycle is an endocardiac acceleration sensor, and
- the means for measuring the haemodynamic parameters comprise:
· means designed to isolate, in the signal delivered by the endocardiac acceleration sensor, at least one component corresponding to an endocardiac acceleration spike; and
· means designed to derive from said component the haemodynamic index (PEA1, PEA2).

6. Device according to Claim 1, comprising means for activating (36, 38; 60, 62) the means for adjusting the vagal stimulation energy at predetermined regular intervals.

7. Device according to Claim 1, comprising:
- means for detecting (34; 58) predetermined changes of state of the patient; and
- means for activating the means for adjusting the vagal stimulation energy upon detection of a change of at least one state of the patient.

8. Device according to Claim 7, in which the predetermined changes of state are changes of the group formed by: presence or absence of arrhythmia, transitions between phases of rest, effort and sleep.

9. Device according to Claim 1, in which the sensor (16) for measuring the haemodynamic parameters of the cardiac cycle is a sensor from the group formed by: endocavitary or epicardiac acceleration sensors; myocardium wall movement sensor; intracardiac pressure sensor; intracardiac bioimpedance sensor; optical measurement oxygen saturation sensor; ultrasound volume variation measurement sensor.

## Patentansprüche

1. Aktive implantierbare medizinische Vorrichtung, die Folgendes enthält:
- Einrichtungen zur vagalen Stimulation (10, 18), die an den Nervus vagus des die Vorrichtung tragenden Patienten wiederholt während aufeinanderfolgender Herzzyklen Impulse liefern können, die einen eingestellten Energiepegel aufweisen;
- einen Sensor (16) zur Messung der hämodynamischen Parameter des Herzzyklus, der einen zeitlichen Parameter liefern kann, der für die Ventrikelfüllzeit repräsentativ ist; und
- Einrichtungen zur Einstellung der vagalen Stimulationsenergie, die wiederholt dynamisch über mehrere Herzzyklen arbeiten und Einrichtungen enthalten, die den Pegel der Stimulationsenergie während aufeinanderfolgender Herzzyklen variieren lassen können (30, 32; 50, 56),
wobei die Vorrichtung **dadurch gekennzeichnet ist, dass**:
- die Einrichtungen, die den Pegel der Stimulationsenergie während aufeinanderfolgender Herzzyklen variieren lassen können, ebenfalls die korrelativen Variationen der Füllzeit (FT1, FT2) bewerten können (26; 46), und
- die Vorrichtung außerdem Einrichtungen enthält, um die Stimulationsenergie auf einem eingestellten Pegel festzulegen, der die Füllzeit maximiert.

2. Vorrichtung nach Anspruch 1, bei der:
- der Sensor (16) zur Messung der hämodynamischen Parameter des Herzzyklus ein Sensor der endokardialen Beschleunigung ist, und
- die Einstelleinrichtungen der vagalen Stimulationsenergie Folgendes enthalten:
· Einrichtungen, die in dem vom Sensor der endokardialen Beschleunigung gelieferten Signal mindestens eine Komponente isolieren können, die einer endokardialen Beschleunigungsspitze entspricht;
· Einrichtungen, die eine morphologische Analyse der Komponente durchführen können; und
· Einrichtungen, die den für die Ventrikelfüllzeit repräsentativen zeitlichen Parameter aus den Ergebnissen der morphologischen Analyse ableiten können.

3. Vorrichtung nach Anspruch 1, bei der die Einrichtungen zur Einstellung der vagalen Stimulationsenergie ebenfalls Folgendes enthalten:
· Einrichtungen (28; 48, 52), um die gemessene Füllzeit mit einer vorbestimmten Schwelle (S) zu vergleichen; und
· Einrichtungen, um den eingestellten Pegel auf den laufenden Pegel der Stimulationsenergie zu begrenzen, sobald die Schwelle erreicht ist.

4. Vorrichtung nach Anspruch 1, bei der:
- der Sensor zur Messung der hämodynamischen Parameter des Herzzyklus ebenfalls einen hämodynamischen Parameter liefern kann, der für die Kontraktilität des linken Ventrikels repräsentativ ist, und
- die Einstelleinrichtungen der vagalen Stimulationsenergie ebenfalls Folgendes enthalten:
· Einrichtungen zur Bewertung (54) der Veränderungen des hämodynamischen Parameters (PEA1, PEA2) während der aufeinanderfolgenden Herzzyklen; und
· Einrichtungen, um den eingestellten Pegel im Fall einer Erhöhung des hämodynamischen Parameters nach einer Erhöhung des Pegels der Stimulationsenergie auf den laufenden Pegel der Stimulationsenergie zu begrenzen.

5. Vorrichtung nach Anspruch 4, bei der:
- der Sensor (16) zur Messung der hämodynamischen Parameter des Herzzyklus ein Sensor der endokardialen Beschleunigung ist, und
- die Einrichtungen zur Messung der hämodynamischen Parameter Folgendes enthalten:
· Einrichtungen, die in dem vom Sensor der endokardialen Beschleunigung gelieferten Signal mindestens eine Komponente isolieren können, die einer endokardialen Beschleunigungsspitze entspricht; und
· Einrichtungen, die von der Komponente den hämodynamischen Index (PEA1, PEA2) ableiten können.

6. Vorrichtung nach Anspruch 1, die Einrichtungen (36, 38; 60, 62) enthält, um die Einstelleinrichtungen der vagalen Stimulationsenergie in vorbestimmten regelmäßigen Abständen zu aktivieren.

7. Vorrichtung nach Anspruch 1, die Folgendes enthält:
- Einrichtungen (34; 58), um vorbestimmte Zustandsänderungen des Patienten zu erfassen; und
- Einrichtungen, um die Einstelleinrichtungen der vagalen Stimulationsenergie bei Erfassung einer Änderung mindestens eines Zustands des Patienten zu aktivieren.

8. Vorrichtung nach Anspruch 7, bei der die vorbestimmten Zustandsänderungen Änderungen der Gruppe sind, die gebildet wird von: Vorhandensein oder Abwesenheit von Arrhythmien; Übergänge zwischen Ruhe-, Anstrengungs- und Schlafphasen.

9. Vorrichtung nach Anspruch 1, bei der der Sensor (16) zur Messung der hämodynamischen Parameter des Herzzyklus ein Sensor der Gruppe ist, die gebildet wird von: einem Sensor der endokavitären oder epikardialen Beschleunigung; einem Bewegungssensor einer Wand des Myokards; einem Sensor des intrakardialen Drucks; einem Sensor der intrakardialen Bioimpedanz; einem Sensor der Sauerstoffsättigung durch optische Messung; einem Sensor der Ultraschall-Volumenänderungsmessung.
